# EUROPEAN PATENT APPLICATION

(11) **EP 3 901 631 A2**
(43) Date of publication of application: **27.10.2021**
(21) Application number: 21174691.2
(22) Date of filing: 16.08.2018
(51) Int. Cl.: G01N 33/68

(54) **MARKERS IN PREPUBERTY FOR CHILDHOOD-PREDIABETES**

(30) Priority: 17.08.2017 EP 17186567
(62) Divisional of application: 18752507.6
(71) Applicant: Société des Produits Nestlé S.A., 1800 Vevey (CH)
(72) Inventor: MARTIN, François-Pierre, 1687 Vuisternens-devant-Romont (CH); COMINETTI, Ornella, 1026 DENGES (CH); PINKNEY, Jonathan, PLYMOUTH, PL4 8AA (GB); HOSKING, Joanne, PLYMOUTH, PL6 8BX (GB)
(74) Representative: Chautard, Cécile

(57) **Abstract**

The present invention generally relates to a method for predicting high blood level glucose in biofluid of a subject. Methods of improving glucose level management in an adolescent subject are also provided.

## Description

The present invention relates to markers in early childhood (prepuberty) for childhood-prediabetes. A metabolic target for nutritional intervention in pre-puberty and adolescence is also provided.

### INTRODUCTION

The rise in chronic and progressive diseases worldwide leads to new challenges in the field of health economics (Nicholson, 2006). The metabolic syndrome encompasses multifactorial metabolic abnormalities including visceral obesity, glucose intolerance, hypertension, hyperuricaemia, dyslipidemia and non-alcoholic fatty liver disease, all of which are associated with an increased long term risk of type 2 diabetes and cardiovascular disease in adults (Mottillo et al., 2010; Scherer et al., 2014). Although insulin resistance (IR) remains a key mechanism underlying the pathophysiology of the metabolic syndrome, many studies support a more complex etiology that also involves genetic factors, body composition, nutrition, and lifestyle. In particular, adiposity has been subject of extensive research, suggesting that its quantitative and qualitative (e.g subcutaneous, visceral) distribution in the body is associated with different risks of cardiovascular disease and abolic and diabetes (Wildman et al., 2008).

New evidence has pointed towards the critical and long-term importance of early life nutrition and lifestyle influences on later health and disease predisposition (Koletzko et al., 1998). The rising prevalence of type 2 diabetes and obesity in children is a growing problem, associated with significant long term risks of vascular complications (Rosenbloom et al., 1999; Marcovecchio and Chiarelli, 2013; Cominetti et al., 2014). Childhood obesity has now risen to epidemic levels worldwide, and in the UK more than a third of children are overweight or obese (Jaarsveld and Gulliford, 2014). Since the development of type 2 diabetes can be delayed or prevented by lifestyle and medical interventions, there is increasing awareness that the early identification of children at risk of developing type 2 diabetes is critical. The term "prediabetes" has been used to define individuals with early hyperglycaemia who are at high risk of progressing to develop type 2 diabetes. Metabolic characteristics of children are strongly influenced by the developmental factors. There is a need, therefore to capture and describe the influence of childhood development on metabolic, clinical and anthropometric parameters into the development of IR. There is a need to define appropriate dietary guidelines and develop a more comprehensive characterization of the influence of environmental factors on the origins and evolution of type 2 diabetes and obesity in childhood (Collino et al., 2012;Martin et al., 2013). As a pre-requisite, there is a needto characterise the biological processes associated with individual health trajectories at the different stages of the life cycle, including the critical pubertal physiological window, which may represent an important period of susceptibility for metabolic dysregulation (Mantovani and Fucic, 2014). Recent analysis from the Earlybird study has demonstrated the important influences on IR of age and gender in puberty (Jeffery S et al. Pediatric Diabetes, 2017 In Press).

Obesity is strongly associated with the development of IR, and there are strong associations between adiposity, IR, impaired glucose regulation and the development of type 2 diabetes in both adults and children. However, not all individuals who are obese develop diabetes and understanding of the underlying mechanisms which link obesity and IR remains incomplete. While it is broadly accepted that diabetes results from the combination of insulin secretory failure and/or IR, the accurate measurement of insulin secretion and IR in humans in-vivo is problematic. The most sensitive methods for such measurements (eg hyperglycaemic clamp, euglycaemic hyperinsulinaemic clamp, or multipoint oral glucose tolerance tests) are not well suited to long term prospective studies with repeat measures and they are generally viewed as far too invasive for repeated use in children. Thus, there is a need for far simpler alternatives.

The identification of novel metabolic biomarkers has the potential not only to more accurately identify individuals at risk of diabetes than simple measures of obesity or the more complex measures of insulin secretion and action, but also to further elucidate the mechanisms by which obesity and IR are linked. A review of previous cross-sectional biomarker studies has shown branched chain (BCAA) and aromatic amino acids (AAA) to be consistently and positively associated with IR, prediabetes and type 2 diabetes independently of adiposity in adults (Guach-Ferre et al, 2016). Meta-analysis of eight prospective studies showed that each study-specific difference of concentrations of amino acids isoleucine, leucine and tyrosine was associated with a 36% increased risk of type 2 diabetes. Similarly, valine was associated with a 35% and phenylalanine with a 26% increase in risk, while glycine and glutamine were inversely associated with risk of type 2 diabetes. These associations have led to the suggestion that derangement of BCAA metabolism may be a contributing factor in the development of IR in obesity. However, there are fewer data in children, in whom IR is much more variable, being particularly dependent upon pubertal development as well as changing body composition and physical activity. Nevertheless, a review of 10 studies in children found that BCAAs and AAAs, along with acylcarnitines, were frequently associated with IR, whilst BCAAs and tyrosine were also associated with increased future metabolic risk in the few studies that included longitudinal follow-up (Zhao et al, 2016). However, most of the studies included in this review were cross-sectional and, in addition, their selective focus was on obese children. Thus, the lack of studies of children of normal weight and of longitudinal design represents an important gap in the evidence. As a result, the regulation of glucose metabolism throughout childhood, including the influences of growth, development, endocrine factors, adiposity and lifestyle, remain poorly defined.

To address this particular evidence gap, the EarlyBird study was designed as a longitudinal cohort study of healthy children with the express intent to investigate the influences of anthropometric, clinical and metabolic processes on glucose control during childhood and adolescence. The EarlyBird cohort is a non-interventional prospective study of 300 healthy UK children followed-up annually throughout childhood. The investigators tackled the challenging task of integrating and correlating the temporal variations of these different data types in the Earlybird childhood cohort from age 5 to age 16, including anthropometric, clinical and serum biomarker (metabonomic) data. Biofluid metabolic profiling has emerged as a robust approach to describing metabolic and nutritional characteristics by monitoring a wide range of biochemical metabolites, reflecting a wide range of molecular regulatory processes. Here a metabonomic approach was applied to serum samples in order to generate new insights into complex metabolic processes during growth and development of children. Proton nuclear magnetic resonance (1H-NMR) spectroscopy of human blood serum enables the monitoring of signals related to lipoprotein-bound fatty acyl groups found in triglycerides, phospholipids and cholesteryl esters, and major low molecular weight molecules present in blood such as amino acids and other major organic acids. These metabonomic data were analysed to assess the association between glucose and individual metabolites, taking into account age, BMI (standard deviation score - sds), physical activity and pubertal timing in a longitudinal fashion.

Recent integration of longitudinal data on insulin, glucose, pubertal onset, age, sex, adiposity, and IGF-1 has highlighted a strong and gender-specific relationship between adiposity, insulin and glucose in childhood, which differs in many ways with the adult phenotype (Jeffery et al., 2012). Growth during childhood and adolescence occurs at different rates and is influenced by the interaction of genetic, nutritional and environmental factors, in turn influencing susceptibility to childhood disease and disease risks later in life. This also introduces a temporal dimension in the study design and poses additional analytical challenges. Although relatively little is known about the underlying genetic regulation, growth variability during puberty correlates with a complex genetic architecture affecting growth, timing of puberty and adiposity (Cousminer et al., 2013). In the context of metabolic health, childhood and adolescence, obesity introduces a significant disturbance into normal growth and pubertal patterns (Sandhu et al., 2006; Marcovecchio and Chiarelli, 2013).

There is evidence in both adults and children that high glucose levels within the normal range (so-called "prediabetes") are indicative of future type 2 diabetes. IR is associated with diabetes and is modulated by complex patterns of external factors throughout childhood. IR is higher during puberty in both males and females, with some studies showing the increase to be independent of changes in adiposity (Jeffery et al., 2012). Modelling of longitudinal data on IR, its relationship to pubertal onset, and interactions with age, sex, adiposity, and IGF-1 has been recently conducted (Jeffery et al., 2012). The study exemplified how IR starts to rise in mid-childhood, some years before puberty, with more than 60% of the variation in IR prior to puberty remaining unexplained. In addition, conventional markers to detect diabetes, and to identify individuals at high risk of developing diabetes, and for adult metabolic disease risk, such as HbA1c, lose sensitivity and specificity for pediatric applications, suggesting that other factors influence the variance of theses markers in youth (Hosking et al., 2014). One potentially important factor currently being studied is the role of excess body weight during childhood, which can also influence pubertal development, through effects on timing of pubertal onset and hormone levels (Marcovecchio and Chiarelli, 2013). The interactions of adiposity with puberty is complex and gender-specific. Moreover, in girls, higher level of IR limit further gain in body fat in the long term - an observation potentially consistent with the concept of IR as a mechanism of insulin desensitization as an adaptive response to weight gain (Hosking et al., 2011).

The complex dynamics of growth and development also involve changes in biological processes that influence basal metabolic function (for instance, resting energy expenditure or REE) and physical activity. The role of resting energy expenditure and weight gain in children is subject to controversy, with particular interest in studying whether low energy expenditure may be a predisposing factor for childhood obesity (Griffiths et al., 1990), and in better understanding of energy requirements prior to and during puberty (Hosking et al., 2010). Recent analysis from the Earlybird study has demonstrated a substantial fall in REE during puberty, independent of adiposity (Mostazir et al. 2016). This suggests that the period of maximum growth is associated with protection of energy reserves, which may have been evolutionarily important, but maladaptive in the current age. These findings also suggest the potential importance of puberty for influencing long term body composition.

In summary, the Earlybird cohort is a uniquely detailed longitudinal cohort study specifically designed to investigate the influences of childhood growth and development on metabolic processes and long term metabolic health risks. The inventors have now undertaken an extensive serum metabonomic analysis in this unique cohort in order to identify novel biomarkers of hyperglycaemia (prediabetes). In the present application, the inventors have applied mixed effects modelling to assess the association between fasting glucose concentration and individual metabolites, taking into account age, BMI sds, physical activity and pubertal timing. From the metabolites having the strongest contribution to childhood blood glucose trajectories, the inventors have found how pre-pubertal metabolic changes are surprisingly informative of fasting blood glucose in late adolescence (e.g. age 16).

### DEFINITIONS

Various terms used throughout the specification are defined as shown below.

The following terms are used throughout the specification to describe the different early life stages of a subject of the invention, particularly a human subject:
- Infant, Newborn: a human subject during the first month after birth;
- Infant: a human subject between 1 and 23 months of age inclusive;
- Child, Preschool: a human subject between the ages of 2 and 5 inclusive;
- Child: a human subject between the ages of 6 and 12 inclusive;
- Prepuberty: age 6 or 7 of a human subject;
- Mid-childhood: age 7 or 8 of a human subject; and
- Adolescent (or adolescence): a human subject between the ages of 13 and 18 inclusive (the corresponding early life stage in other subjects, for example in dogs, would be between the ages 6 months to 18 months inclusive)

The various metabolites mentioned throughout the specification are also known by other names. For example, the metabolite "3-D-hydroxybutyrate" is also known as (R)-(-)-beta-Hydroxybutyric acid; (R)-3-Hydroxybutanoic acid; 3-D-Hydroxybutyric acid; D-3-Hydroxybutyric acid; (R)-(-)-b-Hydroxybutyrate; (R)-(-)-b-Hydroxybutyric acid; (R)-(-)-beta-Hydroxybutyrate; (R)-(-)-β-hydroxybutyrate; (R)-(-)-β-hydroxybutyric acid; (R)-3-Hydroxybutyrate; (R)-3-Hydroxybutanoate; 3-D-Hydroxybutyrate; D-3-Hydroxybutyrate; 3-delta-Hydroxybutyrate; 3-delta-Hydroxybutyric acid; BHIB; D-(-)-3-Hydroxybutyrate; D-beta-Hydroxybutyrate; delta-(-)-3-Hydroxybutyrate; delta-3-Hydroxybutyrate; delta-3-Hydroxybutyric acid; and delta-beta-Hydroxybutyrate.

The metabolite "citrate" is also known as citric acid; 2-Hydroxy-1,2,3-propanetricarboxylic acid; 2-Hydroxytricarballylic acid; 3-Carboxy-3-hydroxypentane-1,5-dioic acid; 2-Hydroxy-1,2,3-propanetricarboxylate; 2-Hydroxytricarballylate; 3-Carboxy-3-hydroxypentane-1,5-dioate; beta-Hydroxytricarballylate; beta-Hydroxytricarballylic acid.

The metabolite "lactate" is also known as L-lactic acid; (+)-Lactic acid; (S)-(+)-Lactic acid; (S)-2-Hydroxypropanoic acid; (S)-2-Hydroxypropionic acid; L-(+)-alpha-Hydroxypropionic acid; L-(+)-Lactic acid; L-(+)-α-hydroxypropionate; (S)-2-Hydroxypropanoate; 1-Hydroxyethane 1-carboxylate; Milk acid; Sarcolactic acid; D-Lactic acid.

The metabolite "asparagine" is also known as L-asparagine; (2S)-2,4-diamino-4-Oxobutanoic acid; (2S)-2-amino-3-Carbamoylpropanoic acid; (S)-2-amino-3-Carbamoylpropanoic acid; (S)-Asparagine; 2-Aminosuccinamic acid; Aspartamic acid; L-2-Aminosuccinamic acid; L-Asparagin; L-Aspartic acid beta-amide; (2S)-2,4-diamino-4-Oxobutanoate; (2S)-2-amino-3-Carbamoylpropanoate; 2-Aminosuccinamate; Aspartamate; L-Aspartamine; L-2,4-diamino-4-Oxobutanoic acid; b2,4-(S)-diamino-4-oxo-Utanoate; Altheine; alpha Amminosuccinamate; Agedoite.

The metabolite "valine" is also known as L-valine; (2S)-2-amino-3-Methylbutanoic acid; 2-amino-3-Methylbutyric acid; L-(+)-alpha-Aminoisovaleric acid; L-alpha-amino-beta-Methylbutyric acid; (2S)-2-amino-3-Methylbutanoate; 2-amino-3-Methylbutyrate; 2-amino-3-Methylbutanoate; (S)-alpha-amino-beta-Methylbutyric acid; (S)-alpha-amino-beta-Methylbutyrate; (S)-2-amino-3-Methylbutyrate; L-α-amino-β-methylbutyric acid.

The metabolite "creatine" is also known as ((amino(imino)Methyl)(methyl)amino)acetic acid; (alpha-methylguanido)Acetic acid; (N-methylcarbamimidamido)Acetic acid; alpha-methylguanidino Acetic acid; Methylglycocyamine; N-(Aminoiminomethyl)-N-methylglycine; N-[(e)-amino(imino)METHYL]-N-methylglycine; N-Amidinosarcosine; N-Carbamimidoyl-N-methylglycine; N-Methyl-N-guanylglycine; (a-methylguanido)acetate; Methylguanidoacetate; [[amino(imino)Methyl](methyl)amino]acetate; (N-methylcarbamimidamido)Acetate.

The term "pre-diabetes" describes a condition in which fasting blood glucose levels are equal or higher than 5.6mmol / L of blood plasma, although not high enough to be diagnosed with type 2 diabetes. Pre-diabetes has no signs or symptoms. People with pre-diabetes have a higher risk of developing type 2 diabetes and cardiovascular (heart and circulation) disease. Without sustained lifestyle changes, including healthy eating, increased activity and losing weight, approximately one in three people with pre-diabetes will go on to develop type 2 diabetes. There are two pre-diabetic conditions:
- Impaired glucose tolerance (IGT) is where blood glucose levels are equal or higher than 5.6mmol / L of blood plasma but not high enough to be classified as diabetes.
- Impaired fasting glucose (IFG) is where blood glucose levels are escalated in the fasting state but not high enough to be classified as diabetes.
- It is possible to have both Impaired Fasting Glucose (IFG) and Impaired Glucose Tolerance (IGT).

As used herein, the term "reference value" can be defined as the average value measured in biofluid samples of a substantially healthy normal glycaemic population. Said population may have an average fasting glucose level of less than 5.6mmol / L. The average age of said population is preferably about the same as that of the subject. The average BMI sds of said population is preferably about the same as that of the subject. The average physical activity level of said population is preferably about the same as that of the subject. Said population may be of substantially the same race as the human subject. Said population may number at least 2, 5, 10, 100, 200, 500, or 1000 individuals. Said population may be substantially the same breed when the subject is a pet.

The term "high levels of glucose" or "high glucose levels" is defined as equal to or higher than 5.6 mmol / L as measured in a biofluid sample of a subject.

The term "biofluid" can be, for example, human blood (particularly human blood serum, human blood plasma), urine or interstitial fluids.

"Overweight" is defined for an adult human as having a BMI between 25 and 30. "Body mass index" or "BMI" means the ratio of weight in kg divided by the height in metres, squared. "Obesity" is a condition in which the natural energy reserve, stored in the fatty tissue of animals, in particular humans and other mammals, is increased to a point where it is associated with certain health conditions or increased mortality. "Obese" is defined for an adult human as having a BMI greater than 30. "Normal weight" for an adult human is defined as a BMI of 18.5 to 25, whereas "underweight" may be defined as a BMI of less than 18.5.Body mass index (BMI) is a measure used to determine childhood overweight and obesity in children and teens. Overweight in children and teens is defined as a BMI at or above the 85th percentile and below the 95th percentile for children and teens of the same age and sex. Obesity is defined as a BMI at or above the 95th percentile for children and teens of the same age and sex. Normal weight in children and teens is defined as a BMI at or above the 5th percentile and below the 85th percentile for children and teens of the same age and sex. Underweight in children and teens is defined as below the 5th percentile for children and teens of the same age and sex. BMI is calculated by dividing a person's weight in kilograms by the square of height in meters. For children and teens, BMI is age- and sex-specific and is often referred to as BMI-for-age. A child's weight status is determined using an age- and sex-specific percentile for BMI rather than the BMI categories used for adults. This is because children's body composition varies as they age and varies between boys and girls. Therefore, BMI levels among children and teens need to be expressed relative to other children of the same age and sex.

The term "subject" is preferably a human subject or can be a pet subject e.g. a cat or a dog.

The term "substantially" is taken to mean 50% or greater, more preferably 75% or greater, or more preferably 90% or greater. The term "about" or "approximately" when referring to a value or to an amount or percentage is meant to encompass variations of in some embodiments ±20%, in some embodiments ±10%, in some embodiments ±5%, in some embodiments ±1%, in some embodiments ±0.5%, and in some embodiments ±0.1% from the specified value, amount or percentage.

### DETAILED DESCRIPTION

The present invention provides a method for predicting high glucose level in biofluid of a subject, said method comprising:
a. (i) determining the levels of 3-D-hydroxybutyrate and one or more of citrate, lactate, and asparagine in the biofluid of said subject; and/or (ii) determining the levels of one or more of 3-D-hydroxybutyrate, valine, and creatine in the biofluid sample of said subject;
b. (i) comparing the ratios of the levels of one or more of 3-D-hydroxybutyrate:citrate, 3-D-hydroxybutyrate:lactate, and 3-D-hydroxybutyrate:asparagine with a reference value; and/or (ii) comparing the levels of one or more of 3-D-hydroxybutyrate, valine, and creatine with a reference value;
c. identifying the subject as being at higher risk of high glucose level if
   (I) the ratios of the levels of one or more of 3-D-hydroxybutyrate:citrate, 3-D-hydroxybutyrate:lactate, and 3-D-hydroxybutyrate:asparagine are higher than the reference value in b(i); and/or
   (II) the levels of one or more of 3-D-hydroxybutyrate, valine, and creatine are higher than the reference value in b(ii).

The present invention further provides a method for predicting high glucose level, particularly high blood glucose level in a subject, said method comprising:
a. (i) determining the levels of 3-D-hydroxybutyrate and one or more of citrate, lactate, and asparagine in a biofluid sample collected from said subject when in prepuberty; and/or (ii) determining the levels of one or more of 3-D-hydroxybutyrate, valine, and creatine in a biofluid sample collected from said subject when in prepuberty;
b. (i) comparing the ratios of the levels of one or more of 3-D-hydroxybutyrate:citrate, 3-D-hydroxybutyrate:lactate, and 3-D-hydroxybutyrate:asparagine with a corresponding reference value; and/or (ii) comparing the levels of one or more of 3-D-hydroxybutyrate, valine, and creatine with a corresponding reference value;
c. identifying the subject as being at higher risk of high glucose level in adolescence if
   (I) the ratios of the levels of one or more of 3-D-hydroxybutyrate:citrate, 3-D-hydroxybutyrate:lactate, and 3-D-hydroxybutyrate:asparagine are higher than the corresponding reference value in b(i); and/or
   (II) the levels of one or more of 3-D-hydroxybutyrate, valine, and creatine are higher than the corresponding reference value in b(ii).

In one embodiment, the method for predicting high blood level glucose in a subject comprises:
a. determining the levels of 3-D-hydroxybutyrate and one or more of citrate, lactate, and asparagine in a biofluid sample collected from said subject when in prepuberty;
b. comparing the ratios of the levels of one or more of 3-D-hydroxybutyrate:citrate, 3-D-hydroxybutyrate:lactate, and 3-D-hydroxybutyrate:asparagine with a corresponding reference value;
c. identifying the subject as being at higher risk of high blood level glucose in adolescence if the ratios of the levels of one or more of 3-D-hydroxybutyrate:citrate, 3-D-hydroxybutyrate:lactate, and 3-D-hydroxybutyrate:asparagine are higher than the corresponding reference value in (b).

In one embodiment, the method for predicting high blood level glucose in a subject comprises:
a. determining the levels of 3-D-hydroxybutyrate and one or more of citrate and asparagine in a biofluid sample collected from said subject when in prepuberty;
b. comparing the ratios of the levels of one or more of 3-D-hydroxybutyrate:citrate, and 3-D-hydroxybutyrate:asparagine with a corresponding reference value;
c. identifying the subject as being at higher risk of high blood level glucose in adolescence if the ratios of the levels of one or more of 3-D-hydroxybutyrate:citrate, and 3-D-hydroxybutyrate:asparagine are higher than the corresponding reference value in (b).

In one aspect, the method for predicting high blood level glucose in a subject comprises:
a. determining the levels of 3-D-hydroxybutyrate and citrate in a biofluid sample collected from said subject when in prepuberty;
b. comparing the ratios of the levels of 3-D-hydroxybutyrate:citrate with a corresponding reference value;
c. identifying the subject as being at higher risk of high blood level glucose in adolescence if the ratios of the levels of one or more of 3-D-hydroxybutyrate:citrate are higher than the corresponding reference value in (b).

In another aspect, the method for predicting high blood level glucose in a subject comprises:
a. determining the levels of 3-D-hydroxybutyrate and lactate in a biofluid sample collected from said subject when in prepuberty;
b. comparing the ratios of the levels of 3-D-hydroxybutyrate:lactate with a corresponding reference value;
c. identifying the subject as being at higher risk of high blood level glucose in adolescence if the ratios of the levels of one or more of 3-D-hydroxybutyrate:lactate are higher than the corresponding reference value in (b).

In another aspect, the method for predicting high blood level glucose in a subject, said method comprising:
a. determining the levels of 3-D-hydroxybutyrate and asparagine in a biofluid sample collected from said subject when in prepuberty;
b. comparing the ratios of the levels of 3-D-hydroxybutyrate:asparagine with a corresponding reference value;
c. identifying the subject as being at higher risk of high blood level glucose in adolescence if the ratios of the levels of one or more of 3-D-hydroxybutyrate:asparagine are higher than the corresponding reference value in (b).

In one embodiment, high blood level glucose corresponds to equal to or higher than 5.6 mmol fasting glucose / litre human blood plasma.

In an alternative embodiment, the present invention provides a method for predicting blood level fasting glucose below 5.6mmol / L in a subject, said method comprising:
a. determining the levels of 3-D-hydroxybutyrate and one or more of citrate, lactate, and asparagine in a biofluid sample collected from said subject when in prepuberty;
b. comparing the ratios of the levels of one or more of 3-D-hydroxybutyrate:citrate, 3-D-hydroxybutyrate:lactate, and 3-D-hydroxybutyrate:asparagine with a corresponding reference value;
c. identifying the subject as being at lower risk of high blood level glucose in adolescence if the ratios of the levels of one or more of 3-D-hydroxybutyrate:citrate, 3-D-hydroxybutyrate:lactate, and 3-D-hydroxybutyrate:asparagine are lower than the corresponding reference value in (b).

In another embodiment, the method for predicting high blood level glucose in a subject comprises:
a. determining the levels of one or more of 3-D-hydroxybutyrate, valine, and creatine in a biofluid sample collected from said subject when in prepuberty;
b. comparing the levels of one or more of 3-D-hydroxybutyrate, valine, and creatine with a corresponding reference value;
c. identifying the subject as being at higher risk of high blood level glucose in adolescence if the levels of one or more of 3-D-hydroxybutyrate, valine, and creatine are higher than the corresponding reference value in (b);

In one aspect, the method for predicting high blood level glucose in a subject comprises:
a. determining the level of 3-D-hydroxybutyrate in a biofluid sample collected from said subject when in prepuberty;
b. comparing the level of 3-D-hydroxybutyrate with a corresponding reference value;
c. identifying the subject as being at higher risk of high blood level glucose in adolescence if the level of 3-D-hydroxybutyrate is higher than the corresponding reference value in (b).

In another aspect, the method for predicting high blood level glucose in a subject comprises:
a. determining the level of valine in a biofluid sample collected from said subject when in prepuberty;
b. comparing the level of valine with a corresponding reference value;
c. identifying the subject as being at higher risk of high blood level glucose in adolescence if the level of valine is higher than the corresponding reference value in (b).

In another aspect, the method for predicting high blood level glucose in a subject comprises:
a. determining the level of creatine in a biofluid sample collected from said subject when in prepuberty;
b. comparing the level of creatine with a corresponding reference value;
c. identifying the subject as being at higher risk of high blood level glucose in adolescence if the level of creatine is higher than the corresponding reference value in (b).

In an alternative embodiment, the present invention provides a method for predicting low blood level glucose in a subject, said method comprising:
a. determining the levels of one or more of 3-D-hydroxybutyrate, valine, and creatine in a biofluid sample collected from said subject when in prepuberty;
b. comparing the levels of one or more of 3-D-hydroxybutyrate, valine, and creatine with a corresponding reference value;
c. identifying the subject as being at lower risk of high blood level glucose in adolescence if the levels of one or more of 3-D-hydroxybutyrate, valine, and creatine are lower than the corresponding reference value in (b).

Preferably, when determining the levels of one or more of 3-D-hydroxybutyrate, valine, and creatine in a biofluid sample and comparing the levels of one or more of 3-D-hydroxybutyrate, valine, and creatine with a corresponding reference value, said biofluid sample is collected from the subject on preferably at least two occasions, wherein said at least two occasions preferably occur on or after the subject's 5^{th} birthday but before the subject's 9^{th} birthday, and are preferably separated by at least a one year interval, preferably as described herein.

In one embodiment, said biofluid sample collections are taken from the subject at age 5 years, 6 years, 7 years, or 8 years separated by at least a one year interval. In one embodiment, said biofluid sample is taken from the subject at age 6 years and at age 7 years.

In one embodiment, said biofluid sample collections are taken from a normal weight subject.

In one embodiment, said biofluid sample collections are taken from a normal weight subject at age 5 years, 6 years, 7 years, or 8 years separated by at least a one year interval. In one embodiment, said biofluid sample is taken from the normal weight subject at age 6 years and at age 7 years.

In one aspect of the invention, the high blood level glucose corresponds to childhood prediabetes.

In one aspect of the invention, the biofluid sample is taken when the subject is age 6 years.

In one aspect of the invention, more than one biofluid sample is taken from said subject in steps a(i) and/or a(ii).

In one aspect of the invention, metabolite measurements are made by NMR (Nuclear Magnetic Resonance). Alternatively, metabolite measurements may be made by mass spectroscopy or by clinical assay.

In one aspect of the invention, the age sub-range of 13 to 16 years is chosen as being representative of adolescence.

In one aspect of the invention, the age 16 years is chosen as being representative of adolescence.

In one aspect of the invention, the reference value is a predetermined standard.

In one aspect of the invention, the biofluid sample is human blood serum.

In one aspect of the invention, high blood level glucose presents in the form of impaired fasting glucose.

In one aspect of the invention, impaired fasting glucose is measured according to the World Health Organisation (WHO) criteria corresponding to a fasting plasma glucose level from 6.1 mmol/l (110 mg/dL) to 6.9 mmol/L (125 mg/dL).

In another aspect of the invention, wherein impaired fasting glucose is measured according to the American Diabetic Association (ADA) criteria corresponding to a fasting plasma glucose level from 5.6 mmol/L (100 mg/dL) to 6.9 mmol/L (125 mg/dL).

The present invention also provides a method of improving glucose level management in an adolescent subject comprising (i) predicting whether said subject has high blood level glucose according to the invention; and (ii) providing a method of modifying the lifestyle of a subject identified as being at higher risk of having high blood level glucose in adolescence, wherein said dietary intervention reduces the glucose level.

In one aspect of the invention, said modification of lifestyle reduces the likelihood or prevents high blood level glucose.

In one aspect of the invention, said modification of lifestyle is provided through prepuberty and puberty.

In one aspect of the invention, said method reduces the likelihood or prevents the onset of one or more metabolic disorders, particularly type 2 diabetes, particularly in early adulthood.

In one aspect of the invention, said modification of lifestyle is provided through prepuberty, puberty, and adolescence.

In one aspect of the invention, the modification in lifestyle in the subject comprises a change in diet, preferably comprising administering at least one nutritional product to the subject that is part of a diet that modulates levels of glucose

In one aspect of the invention, administering at least one nutritional product to the subject that is part of a diet that modulates levels of glucose promotes a reduction in glucose or prevents an increase in glucose levels in the subject.

In one aspect of the invention, the change in diet comprises a decreased consumption of fat and/or an increase in consumption of low fat foods such that not more than 20% of daily calories are obtained from fat.

Low fat foods includes bread and flour, oats, breakfast cereals, wholegrain rice and pasta, fresh, frozen and tinned vegetables and fruits, dried beans and lentils, baked or boiled potatoes, dried fruits, white fish, shellfish, lean wite meat such as chicken and turkey breast without skin, skimmed and smi skimmed milk, cottage or curd cheese, low fat yoghourt, or egg whites. Most adults get 20%-35% of their daily calories from fat. That equates to about 44 to 77 grams of fat a day if 2,000 calories a day are consumed. Low fat foods can also be selected from wholemeal flour and bread, porridge oats, high-fibre breakfast cereals, dried beans and lentils, walnuts, herring, mackerel, sardines, kippers, pilchards, salmon and lean white meat.

In one aspect of the invention, the change in diet comprises a ketogenic type of diet that provides sufficient protein for body growth and repair, and sufficient calories to maintain the correct weight for age and height.

A ketogenic diet may be achieved by excluding high-carbohydrate foods such as starchy fruits and vegetables, bread, pasta, grains and sugar, while increasing the consumption of foods high in fat such as nuts, cream and butter. A variant of the classic diet known as the medium-chain triglycerides (MCT) ketogenic diet uses a form of coconut oil, which is rich in MCTs, to provide around half the calories. As less overall fat is needed in this variant of the diet, a greater proportion of carbohydrate and protein can be consumed, allowing a greater variety of food choices. In one aspect of the invention, the change in diet comprises a change to a ketogenic diet. In one embodiment, a ketogenic diet is the consumption of under 20g of carbohydrates per day.
In one aspect of the invention, the change in diet comprises a change to a Mediterranean diet.
In one embodiment, said Mediterranean diet is higher in fat, that may include intermittent fasting. For instance, in typical Mediterranean countries breakfast may be skipped, and a big lunch may be taken with equal number of calories as breakfast and lunch.
A Mediterranean diet typically contains three to nine servings of vegetables, half to two servings of fruit, one to 13 servings of cereals and up to eight servings of olive oil daily. In one embodiment, it contains approximately not less than 9300 kJ. In one embodiment, it contains not more than 37% as total fat (particularly not less than 18% as monounsaturated and not more than 9% as saturated). In one embodiment, it contains not less than 33 g of fibre per day.
As an example, food type and intake, as well as nutrient content of the Mediterranean diet are described by Davis et al. (Reference Definition of the Mediterranean Diet: A Literature Review, Davis et al., Nutrients, 7(11), 9139-9153, 2015);

In one aspect of the invention, the change in diet comprises a change to a moderate low carbohydrate diet, to maintain or reach normal blood sugar levels throughout the day. In one embodiment, a moderate low carbohydrate diet is the consumption of between 20g to 50g of carbohydrates per day. By comparison, a standard diet is consumption of about 50g to 100g of carbohydrates per day.
In one aspect of the invention, the change in diet comprises a change to a vegan diet. Typically, a vegan diet is well-balanced in macronutrient and micronutrient composition and results in lower average blood sugar levels throughout the day. Vegan diets are plant-based diet regimens that exclude meat, eggs, dairy products, and any other animal-derived foods and ingredients.
In contrast, a vegetarian diet emphasizes plant-based foods but can also include dairy, eggs, honey, and fish. Both vegan and vegetarian diets can be healthful for all life stages with appropriate selection of plant-based foods that adequately meet nutrition requirements for protein, iron, n-3 fatty acids, iodine, zinc, calcium, and vitamin B12. An intermittent vegan diet regimen that is alternated within a habitual, balanced omnivorous diet can also meet these nutritional requirements.

In one aspect of the invention, the change in diet comprises a supplementation of essential nutrients aiming at improving glucose management, such as essential amino acids, lipid and water soluble vitamins, minerals, or a combination of nutrients.

Examples of essential nutrients are amino acids (phenylalanine, valine, threonine, tryptophan, methionine, leucine, isoleucine, lysine, and histidine); fatty acids (alpha-linolenic acid (omega-3 fatty acid) and linoleic acid (omega-6 fatty acid); vitamins (vitamin A, Bs (1-12), Vitamine C, Vitamin D, Vitamin E); minerals such as "major minerals" (calcium, phosphorus, potassium, sodium, chlorine, and magnesium) and "minor minerals" (metals such as iron, zinc, manganese and copper); and conditional nutrients (choline, inositol, taurine, arginine, glutamine and nucleotides).

In one aspect of the invention, the change in diet is associated with physical activity program management. The physical activity program should be adapted to body composition, medical conditions and age of the subjects, aiming at weight loss or weight management, and improvement of body fat mass and lean mass for optimal glucose management outcome.

For instance, the solution may be part of a Physical Activity Program which use all opportunities for students to be physically active, meet the nationally-recommended minutes of physical activity each day (e.g. 60 minutes of moderate to vigorous physically activity each day). For instance, the program may follow public health guidelines for physical activity for children and young people (as an example, National institute for health and care excellence, UK: https://www.nice.org.uk/guidance).

One aspect of the invention further comprises a step of repeating the step of predicting levels of glucose in a subject after modifying the lifestyle of the subject.

The present invention also provides a kit of parts comprising means to measure levels of creatine, citrate, and asparagine in biofluid of a subject in prepuberty.

The present invention also provides a kit of parts comprising means to measure levels of 3-D-hydroxybutyrate, valine, creatine, citrate, lactate, and asparagine in biofluid of a subject in prepuberty.

The present invention also provides the use of a kit of parts according to the invention, to predict a subject in prepuberty of having high glucose levels or developing -prediabetes in adolescence.

### EXAMPLES

### Example 1

### Methods used during the study

### Study Population

The EarlyBird Diabetes Study incorporates a 1995/1996 birth cohort recruited in 2000/2001 when the children were 5 years old (307 children, 170 boys). The collection of data from the Early Bird cohort is composed of several clinical and anthropometric variables measured on an annual basis from the age of 5 to the age of 16. The study was conducted in accordance with the ethics guidelines of the Declaration of Helsinki II; ethics approval was granted by the Plymouth Local Research Ethics Committee (1999), and parents gave written consent and children verbal assent.

### Anthropometric parameters

BMI was derived from direct measurement of height (Leicester Height Measure; Child Growth Foundation, London, U.K.) and weight (Tanita Solar 1632 electronic scales), performed in blind duplicate and averaged. BMI SD scores were calculated from the British 1990 standards.

Physical activity was measured annually from 5 years by accelerometry (Acti-Graph [formerly MTI/CSA]). Children were asked to wear the accelerometers for 7 consecutive days at each annual time point, and only recordings that captured at least 4 days were used.

Resting energy expenditure was measured by indirect calorimetry using a ventilated flow through hood technique (Gas Exchange Measurement, Nutren Technology Ltd, Manchester, UK). Performance tests reportedly show a mean error of 0.3 ± 2.0% in the measurement of oxygen consumption and 1.8 ± 1% in that of carbon dioxide production. Measurements were performed in a quiet thermoneutral room (20°C) after overnight fasting period of at least 6 hours, to minimize any effect attributable to the thermic effect of food. Data were collected for a minimum of 10 minutes and the respiratory quotient (RQ) was calculated as an indicator of basal metabolic rate (BMR).

### Clinical parameters

Peripheral blood was collected annually into EDTA tubes after an overnight fast and stored at -80°C. Insulin resistance (IR) was determined each year from fasting glucose (Cobas Integra 700 analyzer; Roche Diagnostics) and insulin (DPC IMMULITE) (cross-reactivity with proinsulin, 1%) using the homeostasis model assessment program (HOMA-IR), which has been validated in children.

### Serum metabonomics

400 µL of blood serum were mixed with 200 µL of deuterated phosphate buffer solution 0.6 M KH2PO4, containing 1 mM of sodium 3-(trimethylsilyl)-[2,2,3,3-2H4]-1-propionate (TSP, chemical shift reference δH = 0.0 ppm). 550µL of the mixture were transferred into 5 mm NMR tubes.

1H NMR metabolic profiles of serum samples were acquired with a Bruker Avance III 600 MHz spectrometer equipped with a 5 mm cryoprobe at 310 K (Bruker Biospin, Rheinstetten, Germany) and processed using TOPSPIN (version 2.1, Bruker Biospin, Rheinstetten, Germany) software package as reported previously. Standard 1H NMR one-dimensional pulse sequence with water suppression, Carr-Purcell-Meiboom-Gill (CPMG) spin-echo sequence with water suppression, and diffusion-edited sequence were acquired using 32 scans with 98K data-points. The spectral data (from δ 0.2 to δ 10) were imported into Matlab software with a resolution of 22K data-points (version R2013b, the Mathworks Inc, Natwick MA) and normalized to total area after solvent peak removal. Poor quality or highly diluted spectra were discarded from the subsequent analysis.

1H-NMR spectrum of human blood plasma enables the monitoring of signals related to lipoprotein bound fatty acyl groups found in triglycerides, phospholipids and cholesteryl esters, together with peaks from the glyceryl moiety of triglycerides and the choline head group of phosphatidylcholine. This data also covers quantitative profiling of major low molecular weight molecules present in blood. Based on internal database, representative signals of metabolites assignable on 1H CPMG NMR spectra were integrated, including asparagine, leucine, isoleucine, valine, 2-ketobutyric acid, 3-methyl-2-oxovaleric acid, alpha-ketoisovaleric acid, (R)-3-hydroxybutyric acid, lactic acid, alanine, arginine, lysine, acetic acid, N-acetyl glycoproteins, O-acetyl glycoproteins, acetoacetic acid, glutamic acid, glutamine, citric acid, dimethylglycine, creatine, citrulline, trimethylamine, trimethylamine N-oxide, taurine, proline, methanol, glycine, serine, creatinine, histidine, tyrosine, formic acid, phenylalanine, threonine, and glucose. In addition, in diffusion edited spectra, signals associated to different lipid classes were integrated, including phospholipids containing choline, VLDL subclasses, unsaturated and polyunsaturated fatty acid. The signals are expressed in arbitrary unit corresponding to a peak area normalized to total metabolic profiles, which is representative of relative change in metabolite concentration in the serum.

### Statistics

Using data at all ages simultaneously, mixed effects modelling was used to assess the association between glucose and individual metabolites, taking into account age, BMI sds, physical activity and pubertal timing (APHV). Random intercepts were included as well as age (categorized to allow for non-linear change in glucose over time), gender, BMI sds, APHV, MVPA (number of minutes spent in moderate-vigorous physical activity) and individual metabolites (in separate models) as fixed effects. Modelling was carried out in R software using the Imer function in the package Ime4.

### Example 2

### Measurement of metabolite concentrations

**Table 1:**

| | | Glucose (mmol) | | Insulin (mU) | | Moderate-vigorous physical activity (minutes/day) mvpa | | BMI sds | | Respiratory Quotient | |
|---|---|---|---|---|---|---|---|---|---|---|---|
| Age | Gender | mean | sd | mean | sd | mean | sd | mean | sd | mean | sd |
| 6 | M | 4.52 | 0.37 | 3.37 | 3.16 | 61.91 | 23.64 | 0.19 | 0.99 | 0.88 | 0.08 |
| | F | 4.41 | 0.33 | 4.42 | 2.89 | 54.76 | 17.54 | 0.58 | 0.96 | 0.88 | 0.09 |
| 16 | M | 5.17 | 0.33 | 5.59 | 4.40 | 44.48 | 23.73 | 0.51 | 1.13 | 0.95 | 0.15 |
| | F | 5.02 | 0.36 | 6.60 | 5.85 | 32.14 | 22.93 | 0.87 | 1.14 | 0.95 | 0.09 |

Characteristics of the children at age 6 and 16 years are summarized in Table 1. In both genders there was an increase in glucose, insulin, BMI sds, respiratory quotient up to year 16, and a decrease in physical activity as noted with mvpa parameter.

Using data at all ages simultaneously, mixed effects modelling were applied to assess the association between glucose and individual metabolites. The outcome of the models generated for each metabolite is reported in Table 2, for each metabolite pertaining to a given metabolic pathway. Data are reported to statistical significance and alphabetic order for metabolic pathways and metabolites (Table 2).

**Table 2:**

| **Metabolic pathway** | **Metabolite** | **Coef** | **SE** | **p-value** | |
|---|---|---|---|---|---|
| Amino acid derivatives | 2-ketobutyrate | -1245.2 | 129.37 | 0.000000 | x10⁰ |
| Amino acid derivatives | 3-Methyl-2oxovalericacid | -2048.1 | 210.19 | 0.000000 | x10⁰ |
| Branched chain amino acids | Leucine | -148.66 | 11.75 | 0.000000 | x10⁰ |
| Branched chain amino acids | Valine | -132.17 | 13.91 | 0.000000 | x10⁰ |
| Ketone bodies | 3-hydroxybutyrate | -169.3 | 11.4 | 0.000000 | x10⁰ |
| Glycolysis related | Glucose | 55.25 | 6.74 | 8.881784 | x10-16 |
| Glycolysis related | Citrate | -159.49 | 19.94 | 3.996803 | x10-15 |
| Amino acids | Arginine | -240.38 | 32.73 | 4.760636 | x10-13 |
| Branched chain amino acids | Isoleucine | -395.96 | 53.93 | 4.822809 | x10-13 |
| Organic acid | Creatine | -214.45 | 29.5 | 8.073542 | x10-13 |
| Amino acids | Asparagine | -747.56 | 119.7 | 6.68493 | x10-10 |
| Glycolysis related | Glucose | 28.68 | 4.64 | 9.860142 | x10-10 |
| Organic acid | Creatine | -245.27 | 43.25 | 1.931336 | x10-8 |
| Glycolysis related | Lactate | 2.39 | 1.22 | 4.993002 | x10-2 |

The analysis has highlighted the importance of specific metabolites in amino acid, ketone body, glycolysis and fatty acid metabolism, in describing the variations of blood glucose throughout the childhood. This is believed to be the first report of a metabolic contribution of specific metabolic processes to overall blood glucose variations in a longitudinal and continuous manner. The analysis describes how the metabolism of branched chain amino acid and it catabolism, ketogenesis, gluconeogenic amino acids are contributing to glucose production throughout childhood.

The concentrations of these metabolites for boys and girls are reported in Table 3 at each chronological age. The information enables the appreciation of various age-related dynamics in the circulating levels of these metabolites.

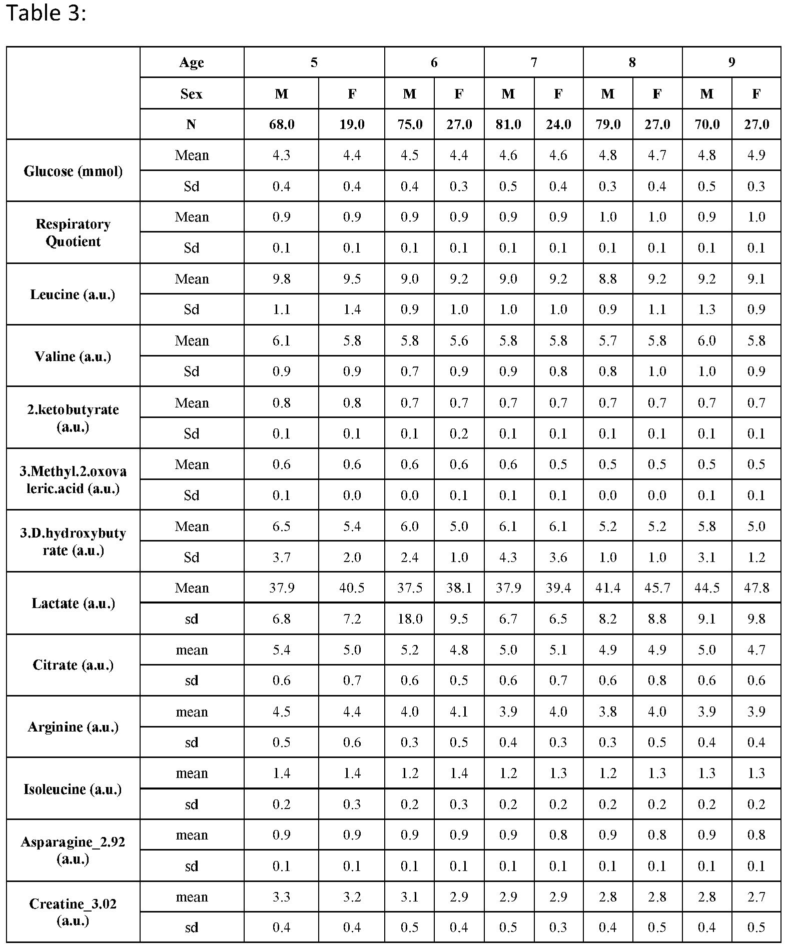

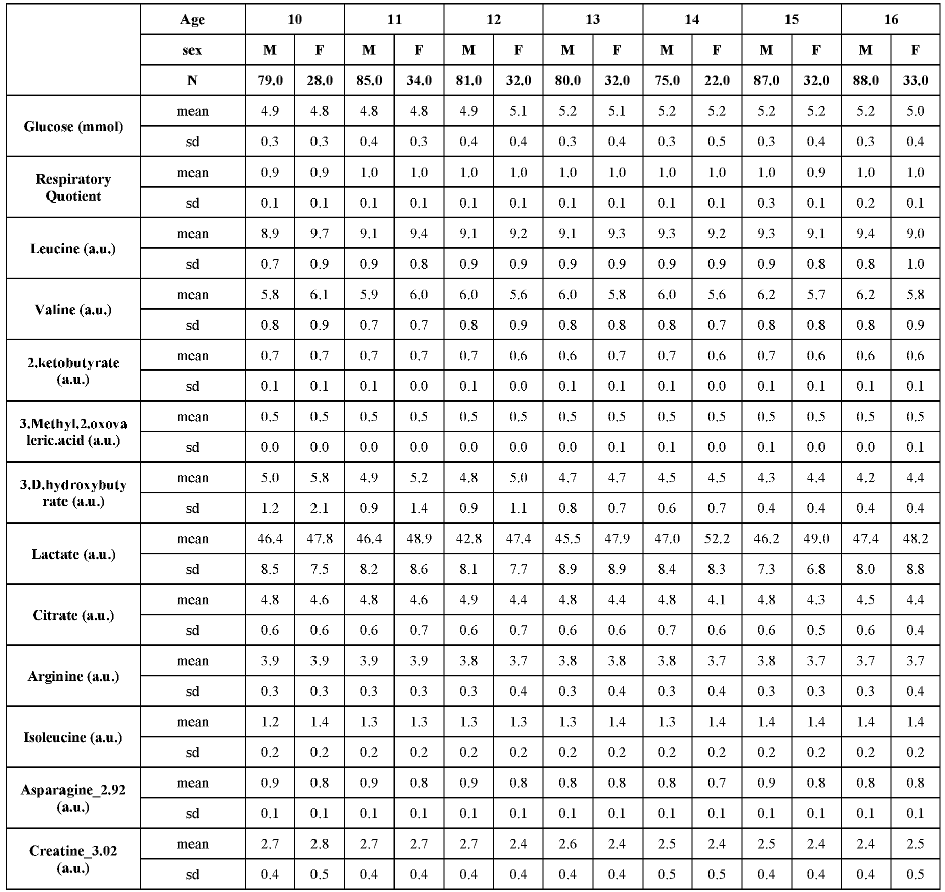

In particular, glucose concentrations show major biological increase in their circulating levels from 4.3 and 4.4 mmol at age 5 to 5.2 and 5.0 mmol at age 16, for boys and girls, respectively. Interestingly, this increased concentration is marked by two plateaus, one between age 8 to 11, and a second one from age 13 to 16. As blood glucose concentrations increase, an increased pattern in the Respiratory Quotient (RQ) with age is seen. This RQ is very informative to reflect basal metabolic rate and which macronutrients are being metabolized for energy fuelling, and therefore as primary energy sources for growth and development during childhood. From age 5 to 7 RQ values remain on average between 0.87 and 0.92, increasing between 0.93 and 0.97 from age 8 to 10, increasing further between 0.98 and 1.01 from age 11 to 13, and decreasing towards 0.95 from year 14 onwards. As reference information, a value of 0.7 indicates that lipids are being metabolized, 0.8 for proteins, and 1.0 for carbohydrates. In other words, when a RQ is equal to one, the body is almost exclusively using endogenous and exogenous carbohydrates as source of metabolic fuels, whilst a value of 0.7 would corresponds to a context of extreme starving where body will use almost exclusively fat as source of metabolic fuels.

There are therefore major changes in glucose production and consumption throughout childhood that are described through the patterns in fasting glucose and RQ. In childhood, such data illustrate that a child may depend exclusively of carbohydrate to fuel it body during a specific period during its puberty (age 11 to 13), and when reaching adolescence during the late stage of puberty, the range of selection for metabolic fuels increases again, more likely towards an adult phenotype. Interestingly, the data shows major changes in prepubertal stage (less than 8 years old) for both boys and girls.

Interestingly, the observation of the changes in the metabolite most associated with glucose variations across childhood, highlighted very particular patterns in 2-ketobutyrate, 3-methyl-2-oxovalerate, leucine, valine, isoleucine, 3-hydroxybutyrate, acetoacetate, citrate, arginine, asparagine and creatine.

Arginine, 2-ketobutyrate and 3-methyl-2-oxovalerate show a decreasing pattern from age 5 to 12, before reaching a plateau. Leucine and Valine remain relative constant over the childhood. Isoleucine shows an interesting pattern with decreased level from age 5 towards a lower and constant concentration from age 6 to 10, followed by an increase up to age 16. Asparagine shows a decreasing pattern from age 5 to 8 in boys and girls, followed by a plateau from age 9 onwards in boys, whereas in girls the metabolite shows a further decrease until age 14 followed by an increase towards the levels seen in boys at age 16. The ketone body 3-d-hydroxybutyrate, creatine and citrate show very high concentrations from age 5 to 7, followed by a steady decrease until age 16.

### Example 3

### Metabolites indicative of higher blood sugar at adolescence

Based on the above observations, it was further explored - amongst the metabolites contributing the most to glucose variations in childhood - which ones may be an earlier and a more indicative indicator of higher blood sugar at adolescence. In Table 4, the values of blood glucose in children from age 5 to 16 are reported for children classified as normoglycemic or with impaired fasting glycemia at age 16 (e.g. fasting glucose above ADA criteria, i.e. equal or greater than 5.6mmol / L of plasma). The glucose state at year 16 is representative of glucose stage at years 13, 14, and 15. Both groups of children show similar concentrations of blood glucose during pre-puberty, whilst differences in their fasting blood concentration of glucose is detectable only during adolescence.

**Table 4:**

| | Age | Gender | Glucose (mmol / L) | sd |
|---|---|---|---|---|
| Normoglycemic | 5 | M | 4.3 | 0.4 |
| | 5 | F | 4.4 | 0.4 |
| | 6 | M | 4.5 | 0.4 |
| | 6 | F | 4.4 | 0.3 |
| | 7 | M | 4.6 | 0.5 |
| | 7 | F | 4.6 | 0.4 |
| | 8 | M | 4.8 | 0.3 |
| | 8 | F | 4.7 | 0.4 |
| | 9 | M | 4.7 | 0.5 |
| | 9 | F | 4.9 | 0.3 |
| | 10 | M | 4.9 | 0.3 |
| | 10 | F | 4.8 | 0.2 |
| | 11 | M | 4.8 | 0.3 |
| | 11 | F | 4.8 | 0.3 |
| | 12 | M | 4.9 | 0.4 |
| | 12 | F | 5.1 | 0.4 |
| | 13 | M | 5.1 | 0.3 |
| | 13 | F | 5.1 | 0.4 |
| | 14 | M | 5.2 | 0.3 |
| | 14 | F | 5.1 | 0.5 |
| | 15 | M | 5.1 | 0.3 |
| | 15 | F | 5.2 | 0.4 |
| | 16 | M | 5.1 | 0.3 |
| | 16 | F | 4.9 | 0.3 |
| IFG | 5 | M | 4.5 | 0.4 |
| | 5 | F | 4.3 | 0.1 |
| | 6 | M | 4.5 | 0.4 |
| | 6 | F | 4.4 | 0.6 |
| | 7 | M | 4.9 | 0.3 |
| | 7 | F | 4.7 | 0.5 |
| | 8 | M | 4.9 | 0.4 |
| | 8 | F | 4.4 | 0.7 |
| | 9 | M | 5.1 | 0.3 |
| | 9 | F | 5.0 | 0.3 |
| | 10 | M | 5.0 | 0.4 |
| | 10 | F | 4.7 | 0.4 |
| | 11 | M | 5.1 | 0.3 |
| | 11 | F | 4.8 | 0.4 |
| | 12 | M | 5.2 | 0.3 |
| | 12 | F | 4.9 | 0.5 |
| | 13 | M | 5.5 | 0.1 |
| | 13 | F | 5.1 | 0.5 |
| | 14 | M | 5.5 | 0.3 |
| | 14 | F | 5.4 | 0.4 |
| | 15 | M | 5.5 | 0.2 |
| | 15 | F | 5.3 | 0.4 |
| | 16 | M | 5.7 | 0.1 |
| | 16 | F | 5.6 | 0.0 |

Therefore, it was assessed if some of the key metabolites associated with glucose trajectories in childhood may be providing more sensitive information with regards to blood glucose at age 16. To achieve this objective, a strategy was adopted aimed at comparing the correlation coefficients generated between blood glucose at year 16 and the ratio of changes of influential metabolites between age 5 to 6, 6 to 7, and 7 to 8, respectively. Most significant associations were identified from the metabolite variations between age 6 and 7. The results are reported in Table 5. As a reference, the results were compared with the correlations obtained between glucose at year 16 and changes in BMI sds from age 6 to 7, or between glucose at year 16 and changes in glucose from age 6 to 7. Amongst the most influential metabolites, 3-D-hydroxybutyrate, valine and creatine had their yearly variations statistically and positively associated to glucose concentration at year 16, and more informative than BMI sds or glucose alone.

Considering the previous results, it was further tested if a ratio between metabolites at age 6 or 7 would be as informative as a temporal variation in a given metabolite. Due to the statistical and biological relationship between 3-hydroxybutyrate and glucose, it was decided to compute the ratio between metabolites and 3-D-hydroxybutyrate, and assess the correlations with blood glucose at year 16. The results are also reported in Table 5. Very interestingly, it was seen that the ratios 3-D-hydroxybutyrate/ citrate, 3-D-hydroxybutyrate/ lactate, 3-D-hydroxybutyrate/ asparagine at year 6 provide a good correlation with the blood glucose at year 16, statistically better than 3-D-hydroxybutyrate/ glucose ratio. The analysis also shows that 3-D-hydroxybutyrate/ valine or 3-D-hydroxybutyrate/ creatine have much lower relationships to blood glucose at year 16, indicating these 3 metabolites are closely biologically related.

**Table 5:**

| **Parameter** | Pearson Correlation with Glucose Age 16 for ratio of parameters age 6/ Age 7 | p-val | Least squares regression slope | Pearson Correlation with Glucose Age 16 for Ratio between 3-D-hydroxybutyrate at age 6/ Variable at age Age 6 | p-val | Least squares regression slope |
|---|---|---|---|---|---|---|
| BMI | -0.05 | 0.6832 | -0.01239 | NA | NA | NA |
| Glucose | -0.2 | 0.072 | -0.6899 | 0.23 | 0.0279 | 123.197 |
| **Citrate** | 0.08 | 0.4742 | 0.176 | **0.26** | **0.0093** | **0.2418** |
| Lactate | -0.03 | 0.8086 | -0.01875 | **0.21** | **0.0439** | **1.05** |
| **Acetate** | 0.02 | 0.8439 | 0.03419 | **0.32** | **0.0017** | **0.08692** |
| Acetoacetate | 0.27 | 0.0145 | 0.5274 | 0.24 | 0.0193 | 0.0713 |
| **Asparagine** | 0.02 | 0.827 | 0.06048 | **0.27** | **0.0084** | **0.03951** |
| Leucine | 0.13 | 0.2417 | 0.4048 | 0.26 | 0.0108 | 0.09448 |
| **3-D-hydroxybutyrate** | **0.34** | **0.0019** | **0.2208** | **NA** | **NA** | **NA** |
| **Valine** | **0.36** | **8.00E-04** | **0.7996** | 0.16 | 0.127 | 0.161 |
| 3-Methyl-2oxovalericacid | 0.17 | 0.1163 | 0.5528 | 0.26 | 0.0104 | 0.02807 |
| 2-Ketobutyrate | 0.1 | 0.3481 | 0.3481 | 0.24 | 0.02 | 0.03241 |
| Arginine | 0.24 | 0.0257 | 0.9175 | 0.26 | 0.0118 | 0.1717 |
| **Creatine** | **0.34** | **0.0017** | **0.7361** | 0.19 | 0.0673 | 0.0979 |
| Isoleucine | 0.18 | 0.1081 | 0.321 | 0.21 | 0.0435 | 0.03955 |
| PUFA | 0.26 | 0.0191 | 1.019 | 0.22 | 0.0342 | 0.08206 |
| Lysine | -0.05 | 0.6688 | -0.1513 | 0.26 | 0.0104 | 0.1783 |
| **Trimethylamine** | -0.07 | 0.5115 | -0.2176 | 0.27 | 0.0084 | 0.03951 |
| Methyl.Lipid signal | 0.28 | 0.0106 | 0.931 | 0.21 | 0.0441 | 1.751 |
| **Formate** | -0.09 | 0.4429 | -0.1948 | 0.29 | 0.0046 | 0.09063 |
| **Histidine** | -0.09 | 0.4429 | -0.1948 | 0.29 | 0.0046 | 0.09063 |
| Serine | 0.05 | 0.6639 | 0.1495 | 0.24 | 0.0171 | 0.1317 |

Therefore, amongst the most influential biochemical species contributing to high fasting glucose in childhood, the analysis indicates that:
- The measure of 3-D-hydroxybutyrate, valine, and creatine at age 6 and 7 are key indicators of high glucose at year 16, and therefore of risk of IFG
- The measure of 3-D-hydroxybutyrate/ citrate, or 3-D-hydroxybutyrate/ lactate, or 3-D-hydroxybutyrate/ asparagine at year 6 is a key indicator of high glucose at year 16, and therefore of risk of IFG
- As an example in the present study cohort, children were classified as impaired fasting glycemia at age 16, once their fasting glucose value are higher than 5.6 mmol.L-1. On average IFG children at year 16 have 11% higher fasting blood glucose than normo glycemic children. Therefore, for the pre-pubertal markers in this study, as an example, it can be stated that:
   ∘ A value of glucose at year 16 which is 11% greater than the average corresponds, on average, to an increase of 205% on the 3-HB/citrate ratio at year 6.
   ∘ A value of glucose at year 16 which is 11% greater than the average corresponds, on average, to an increase of 214% on the 3-HB/acetate ratio at year 6.
   ∘ A value of glucose at year 16 which is 11% greater than the average corresponds, on average, to an increase of 218% on the 3-HB/asparagine ratio at year 6.
   ∘ A value of glucose at year 16 which is 11% greater than the average corresponds, on average, to an increase of 328% on the 3-HB/lactate ratio at year 6.
   ∘ A value of glucose at year 16 which is 11% greater than the average corresponds, on average, to an increase of 229% on the 3-HB yr 6/3-HB yr 7 ratio.
   ∘ A value of glucose at year 16 which is 11% greater than the average corresponds, on average, to an increase of 69% on the valine yr 6/valine yr 7 ratio.
   ∘ A value of glucose at year 16 which is 11% greater than the average corresponds, on average, to an increase of 73% on the creatine yr 6/creatine yr 7 ratio.
      - Significant increases in the annual incidence of both type 1 diabetes and type 2 diabetes among youths (aged 10 to 19 years old) in the United States have been recently reported by Mayer-Davis et al. (Incidence Trends of Type 1 and Type 2 Diabetes among Youths, 2002-2012, The New England Journal of Medicine, 376:1419-1429, 2017). It is well established that variations exist across racial and ethnic groups. As illustred by Mayer-Davis et al, this includes high relative increases in the incidence of type 2 diabetes in racial and ethnic groups other than non-Hispanic whites in the USA as an example. Variation across demographic subgroups may reflect varying combinations of genetic, environmental, and behavioral factors that contribute to diabetes. Therefore, reference values should be generated accordingly for the proposed markers.

As an example in the present study cohort, reference values are determined from the normoglycemic population (Table 6).

**Table 6:**

| Marker | mean | sd |
|---|---|---|
| 3-HB/citrate ratio at year 6 | 1.111 | 0.339 |
| 3-HB/acetate ratio at year 6 | 2.961 | 1.189 |
| 3-HB/asparagine ratio at year 6 | 6.382 | 2.096 |
| 3-HB/lactate ratio at year 6 | 0.161 | 0.068 |
| 3-HB yr 6/3-HB yr 7 ratio | 1.066 | 0.458 |
| valine yr 6/valine yr 7 ratio | 0.988 | 0.145 |
| creatine yr 6/creatine yr 7 ratio | 1.035 | 0.152 |

## Claims

1. A method for predicting high blood level glucose in a subject, said method comprising:
a. (i) determining the levels of 3-D-hydroxybutyrate and one or more of citrate, lactate, and asparagine in a biofluid sample collected from said subject when in prepuberty; and/or (ii) determining the levels of one or more of 3-D-hydroxybutyrate, valine, and creatine in a biofluid sample collected from said subject when in prepuberty;
b. (i) comparing the ratios of the levels of one or more of 3-D-hydroxybutyrate:citrate, 3-D-hydroxybutyrate:lactate, and 3-D-hydroxybutyrate:asparagine with a corresponding reference value; and/or (ii) comparing the levels of one or more of 3-D-hydroxybutyrate, valine, and creatine with a corresponding reference value;
c. identifying the subject as being at higher risk of high blood level glucose in adolescence if
(I) the ratios of the levels of one or more of 3-D-hydroxybutyrate:citrate, 3-D-hydroxybutyrate:lactate, and 3-D-hydroxybutyrate:asparagine are higher than the corresponding reference value in b(i); and/or
(II) the levels of one or more of 3-D-hydroxybutyrate, valine, and creatine are higher than the corresponding reference value in b(ii).

2. The method for predicting high blood level glucose in a subject according to claim 1, said method comprising:
a. determining the levels of 3-D-hydroxybutyrate and one or more of citrate, lactate, and asparagine in a biofluid sample collected from said subject when in prepuberty;
b. comparing the ratios of the levels of one or more of 3-D-hydroxybutyrate:citrate, 3-D-hydroxybutyrate:lactate, and 3-D-hydroxybutyrate:asparagine with a corresponding reference value;
c. identifying the subject as being at higher risk of high blood level glucose in adolescence if the ratios of the levels of one or more of 3-D-hydroxybutyrate:citrate, 3-D-hydroxybutyrate:lactate, and 3-D-hydroxybutyrate:asparagine are higher than the corresponding reference value in (b).

3. The method for predicting high blood level glucose in a subject according to claim 1, said method comprising:
a. determining the levels of 3-D-hydroxybutyrate and one or more of citrate and asparagine in a biofluid sample collected from said subject when in prepuberty;
b. comparing the ratios of the levels of one or more of 3-D-hydroxybutyrate:citrate, and 3-D-hydroxybutyrate:asparagine with a corresponding reference value;
c. identifying the subject as being at higher risk of high blood level glucose in adolescence if the ratios of the levels of one or more of 3-D-hydroxybutyrate:citrate, and 3-D-hydroxybutyrate:asparagine are higher than the corresponding reference value in (b).

4. The method for predicting high blood level glucose in a subject according to claim 1, said method comprising:
a. determining the levels of one or more of 3-D-hydroxybutyrate, valine, and creatine in a biofluid sample collected from said subject when in prepuberty;
b. comparing the levels of one or more of 3-D-hydroxybutyrate, valine, and creatine with a corresponding reference value;
c. identifying the subject as being at higher risk of high blood level glucose in adolescence if the levels of one or more of 3-D-hydroxybutyrate, valine, and creatine are higher than the corresponding reference value in (b);

5. The method according to any previous claim, wherein the high blood level glucose corresponds to childhood prediabetes.

6. The method according to any previous claim, wherein the biofluid sample is collected when the subject is age 6 or 7 years.

7. The method according to claim 6, wherein the biofluid sample is collected when the subject is age 6 years.

8. The method according to any previous claim, wherein more than one biofluid sample is collected from said subject in steps a(i) and/or a(ii).

9. The method according to any previous claim, wherein adolescence corresponds to age 13 to 16 years.

10. The method according to claim 9, wherein adolescence corresponds to age 16 years.

11. The method according to any previous claim, wherein the biofluid sample is collected from a normal weight subject.

12. The method according to any previous claim, wherein the reference value is a predetermined standard.

13. The method according to any previous claim, wherein the biofluid sample is human blood serum.

14. The method according to any previous claim, wherein high blood level glucose presents in the form of impaired fasting glucose.

15. The method according to claim 14, wherein impaired fasting glucose is measured according to the World Health Organisation (WHO) criteria corresponding to a fasting plasma glucose level from 6.1 mmol/l (110 mg/dL) to 6.9 mmol/L (125 mg/dL).

16. The method according to claim 14, wherein impaired fasting glucose is measured according to the American Diabetic Association (ADA) criteria corresponding to a fasting plasma glucose level from 5.6 mmol/L (100 mg/dL) to 6.9 mmol/L (125 mg/dL).

17. A method of improving glucose level management in an adolescent subject comprising (i) predicting the likelihood of said subject having high blood level glucose according to claims 1 to 16; and (ii) providing a method of modifying the lifestyle of a subject identified as being at higher risk of having high blood level glucose in adolescence, wherein said dietary intervention reduces the glucose level.

18. The method according to claim 17, wherein said modification of lifestyle reduces the likelihood or prevents high blood level glucose.

19. The method according to claim 18, wherein said modification of lifestyle is provided through prepuberty and puberty.

20. The method according to claims 17 to 19, wherein said method reduces the likelihood or prevents type 2 diabetes in adulthood.

21. The method according to claim 20, wherein said modification of lifestyle is provided through prepuberty, puberty, and adolescence.

22. The method according to claims 17 to 21, wherein the modification in lifestyle of the subject comprises a change in diet, preferably comprising administering at least one nutritional product to the subject that is part of a diet that modulates levels of glucose.

23. The method according to claim 22, wherein administering at least one nutritional product to the subject that is part of a diet that modulates levels of glucose, promotes a reduction in glucose or prevents an increase in glucose.

24. The method of claims 22 or 23, wherein the change in diet comprises a decreased consumption of fat and/or an increase in consumption of low fat foods such that, on average, not more than 20% of daily calories are obtained from fat.

25. The method of claim 24 wherein the low fat foods are selected from wholemeal flour and bread, porridge oats, high-fibre breakfast cereals, wholegrain rice and pasta, vegetables and fruit, dried beans and lentils, baked potatoes, dried fruit, walnuts, white fish, herring, mackerel, sardines, kippers, pilchards, salmon and lean white meat.

26. The method of claim 22, wherein the change in diet comprises a ketogenic type of diet, that provides sufficient protein for body growth and repair, and sufficient calories to maintain the correct weight for age and height, wherein said ketogenic diet is the consumption of under 20g of carbohydrates per day.

27. The method of claim 22, wherein the change in diet comprises a Mediterranean diet.

28. The method of claim 22, wherein the change in diet comprises a moderate low carbohydrate diet, that provides a lower average blood sugar levels throughout the day, wherein said moderate low carpbohydrate diet is the consumption of between 20g to 50g of carbohydrates per day.

29. The method of claim 22, wherein the change in diet comprises a well-balanced vegan diet, that provides a lower average blood sugar levels throughout the day.

30. The method of claim 22, wherein the change in diet comprises a supplementation of essential nutrients aiming at improving glucose management, such as essential amino acids, lipid and water soluble vitamins, minerals, or a combination of nutrients.

31. The method of claims 22 to 30, wherein the change in diet is associated with physical activity program management.

32. The method of claims 17 to 31, comprising a further step of repeating the step of predicting levels of glucose in childhood in a subject after modifying the lifestyle of the subject.

33. A kit of parts comprising means to measure levels of 3-D-hydroxybutyrate, valine, creatine, citrate, lactate, and asparagine in a biofluid sample collected from a subject in prepuberty.

34. Use of a kit of parts according to claim 33, to predict a subject in prepuberty of high blood level glucose or developing childhood-prediabetes in adolescence.
